# EUROPÄISCHE PATENTSCHRIFT

(11) **EP 0 673 939 B1**
(45) Veröffentlichungstag und Bekanntmachung des Hinweises auf die Patenterteilung: **23.10.1996**
(21) Anmeldenummer: 95810164.4
(22) Anmeldetag: 13.03.1995
(51) Int. Cl.: C07D 487/04, G03G 5/06

(54) **Aminoxidgruppen enthaltende Pyrrolo[3,4-c]pyrrole als Photorezeptoren**
Aminoxide groups containing pyrrolo(3,4-c)pyrroles as photoreceptors
Pyrrolo(3,4-c)pyrroles contenant des groupes amine-oxyde comme photorécepteurs

(30) Priorität: 21.03.1994 CH 843/94
(43) Veröffentlichungstag der Anmeldung: 27.09.1995
(73) Patentinhaber: CIBA-GEIGY AG, 4002 Basel (CH)
(72) Erfinder: Hao, Zhimin, Dr., CH-1723 Marly (CH); Iqbal, Abul, Dr., CH-1732 Arconciel (CH); Kirchmayr, Rudolf, Dr., CH-1723 Marly (CH)

(56) Entgegenhaltungen:
- EP-A- 0 061 426
- EP-A- 0 094 911
- EP-A- 0 133 156
- EP-A- 0 187 620
- EP-A- 0 612 747
- DATABASE WPI Derwent Publications Ltd., London, GB; AN 90-86701 & JP-A-02 039 159 (FUJI PHOTO FILM K. K.) , 8.Februar 1990
- DATABASE WPI Derwent Publications Ltd., London, GB; AN 91-062038 & JP-A-03 011 357 (DAINIPPON INK CHEM. K. K.) , 18.Januar 1991
- DATABASE WPI Derwent Publications Ltd., London, GB; AN 93-137476 & JP-A-05 072 769 (MITA IND. CO., LTD.) , 26.März 1993

## Beschreibung

Die vorliegende Erfindung betrifft mit heteroaromatischen Aminoxidresten substituierte Diketopyrrolo[3,4-c]pyrrole, ein Verfahren zu ihrer Herstellung und ihre Verwendung als Pigmente und insbesondere als photoleitfähige Substanzen in elektrophotographischen Photorezeptoren.

Aus den US-Patenten 4 579 949 und 4 778 899 sind symmetrisch und asymmetrisch mit Heteroaromaten (spezifisch Pyridyl) substituierte Diketopyrrolo[3,4-c]pyrrole und ihre Verwendung als Pigmente bekannt.

Es sind nun neue, mit heteroaromatischen Aminoxidresten substituierte Diketopyrrolo-[3,4-c]pyrrole gefunden worden, die sehr gute Pigmenteigenschaften aufweisen, sich zudem aber, dank einer überraschend hohen Photoleitfähigkeit, als Photoleiter in elektrophotographischen Photorezeptoren eignen.

Die vorliegende Erfindung betrifft demnach Pyrrolo[3,4-c]pyrrole der Formel 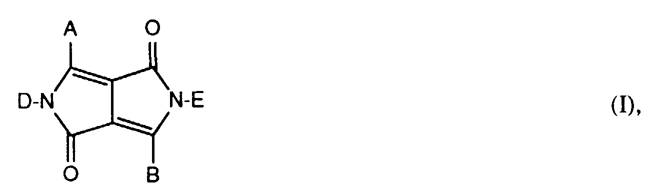 worin A und B unabhängig voneinander für eine Gruppe der Formel 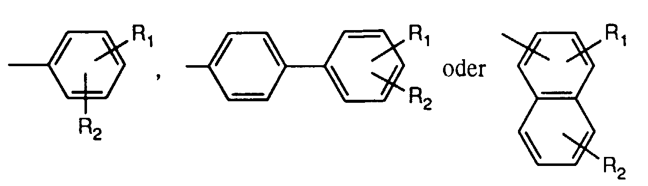 oder für Q stehen, worin
Q einen unsubstituierten oder ein-, zwei- oder dreimal durch C₁-C₁₈-Alkyl, C₁-C₁₈-Alkoxy oder 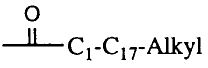 substituierten 5- oder 6-gliedrigen heterocyclischen aromatischen Aminoxidrest ohne oder mit einem oder zwei ankondensierten Benzolringen bedeutet,
R₁ und R₂ unabhängig voneinander Wasserstoff, Halogen, C₁-C₁₈-Alkyl, C₁-C₁₈-Alkoxy, C₁-C₁₈-Alkylmercapto, -CN, -NO₂ oder Trifluormethyl sind,
D und E unabhängig voneinander Wasserstoff, C₁-C₁₈-Alkyl, C₂-C₄-Alkenyl, C₇-C₁₀-Aralkyl, unsubstituiertes oder durch Chlor, Brom, C₁-C₆-Alkyl, C₁-C₄-Alkoxy, Trifluormethyl oder Nitro substituiertes Phenyl bedeuten,
mit der Massgabe, dass mindestens einer der Sustituenten A und B einen Aminoxidrest Q bedeutet.

C₁-C₁₈-Alkylgruppen sind geradkettig oder verzweigt und weisen bevorzugt 1 bis 6 und insbesondere 1 bis 4 C-Atome auf. Es handelt sich dabei z.B. um Methyl, Ethyl, n-Propyl, Isopropyl, n-Butyl, sec.-Butyl, tert.-Butyl, Pentyl, Hexyl, Heptyl, Octyl, Nonyl, Decyl, Undecyl, Tridecyl, Hexadecyl oder Octadecyl.

Beispiele für Alkoxy sind insbesondere Methoxy, Ethoxy, n-Propoxy, Isopropoxy, Butoxy, Pentoxy, Hexyloxy, aber auch Heptyloxy, Octyloxy, Nonyloxy, Decyloxy, Dodecyloxy, Hexydecyloxy oder Octadecyloxy.

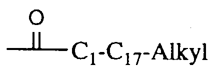

bedeutet z.B. Methyl-, Ethyl-, Propyl-, Pentyl-, Hexyl-, Heptyl-, Hendecyl- oder Heptadecylcarbonyl.

C₁-C₁₈-Alkylmercapto steht beispielsweise für Methyl-, Ethyl-, Propyl-, Butyl-, Octyl-, Decyl-, Hexadecyl- oder Octadecylmercapto.

C₂-C₄-Alkenyl bedeutet z.B. Vinyl, Allyl, Methallyl oder 2-Butenyl.

C₇-C₁₀-Aralkyl steht z.B. für 1-Phenethyl, 1,1-Dimethylbenzyl, im Benzolkern durch Methyl oder Ethyl substituiertes Benzyl oder vor allem für Benzyl.

Bedeuten etwaige Substituenten Halogen, dann handelt es sich z.B. um Jod, Fluor, insbesondere Brom und bevorzugt Chlor.

Von besonderem Interesse sind Pyrrolo[3,4-c]pyrrole der Formel (I) gemäss der oben angegebenen Definition, worin Q ein Rest der Formel 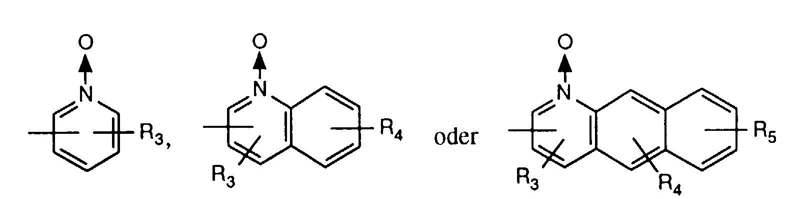 ist, worin R₃, R₄ und R₅ unabhängig voneinander Wasserstoff, C₁-C₄-Alkyl, C₁-C₄-Alkoxy oder 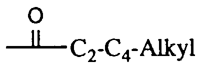 bedeuten.

Bevorzugt werden Pyrrolo[3,4-c]pyrrole der Formel 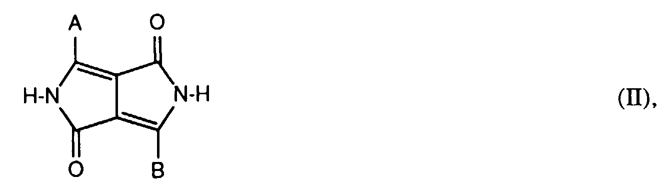 worin A und B unabhängig voneinander für eine Gruppe der Formel 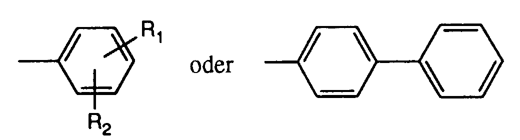 oder für Q stehen, worin
R₁ und R₂ unabhängig voneinander Wasserstoff, Chlor, Brom, C₁-C₄-Alkyl, C₁-C₆-Alkoxy oder CN bedeuten und Q die in der soeben erwähnten Bevorzugung angegebenen Bedeutung hat, insbesondere aber wenn Q ein Rest der Formel 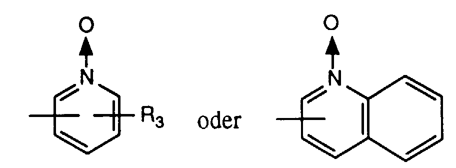 ist, worin R₃ Wasserstoff oder C₁-C₄-Alkyl bedeutet.

Insbesondere bevorzugt werden Pyrrolo[3,4-c]pyrrole der Formel 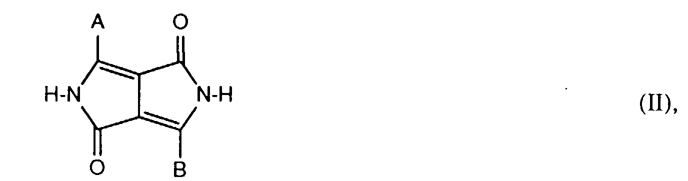 worin A und B unabhängig voneinander für eine Gruppe der Formel 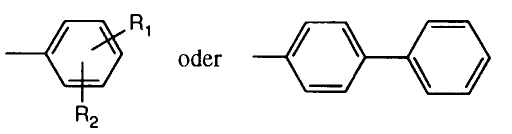 oder für Q stehen, worin
R₁ und R₂ unabhängig voneinander Wasserstoff, Chlor, Brom, C₁-C₄-Alkyl,
C₁-C₆-Alkoxy oder CN bedeuten und Q ein Rest der Formel 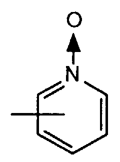 ist.

Pyrrolo[3,4-c]pyrrole der Formel I können in Analogie zu allgemein bekannten Methoden hergestellt werden, z.B.
a) durch Umsetzung von 1 Mol eines Bernsteinsäurediesters der Formel 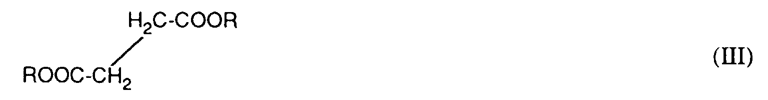 mit je einem Mol der Nitrile 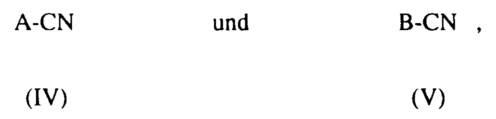 worin R C₁-C₆-Alkyl ist und A und B die oben angegebene Bedeutung haben, wobei mindestens einer der Reste A und B Q sein muss,
   gemäss der im US-Patent 4 579 949 beschriebenen Methode,
   zum Diketopyrrolo[3,4-c]pyrrol der Formel 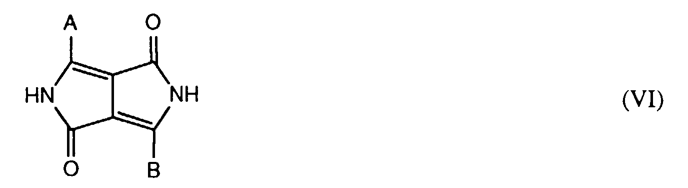 oder
b) durch Umsetzung von 1 Mol des Pyrrolinons der Formel 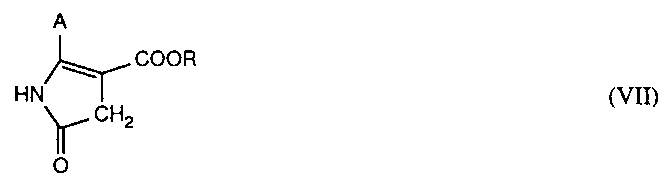 mit einem Mol eines Nitrils der Formel V, worin R, A und B die oben angegebene Bedeutung haben, wobei mindestens einer der Reste A und B Q sein muss,
   gemäss der im US-Patent 4 778 899 beschriebenen Methode,
   ebenfalls zum Diketopyrrolo[3,4-c]pyrrol der Formel VI.
   Aus letzterem kann ein Diketopyrrolo[3,4-c]pyrrol der Formel I, worin D und E nicht Wasserstoff bedeuten z.B. durch Umsetzung von 1 Mol eines Diketopyrrolo[3,4-c]-pyrrols der Formel VI mit je einem Mol einer Verbindung enthaltend die Reste D und E mit der oben ausser Wasserstoff angegebenen Bedeutung als Abgangsgruppen,
   gemäss der in US-Patent 4 585 878 beschriebenen Methode,
   erhalten werden.
   Die durch die Anwesenheit mindestens eines aromatischen Aminoxidrests gekennzeichneten Pyrrolo[3,4-c]pyrrole der Formel I können aber auch
c) durch Oxidation nach allgemein bekannten Methoden eines mindestens einen entsprechenden aromatischen Aminrest enthaltenden Pyrrolo[3,4-c]pyrrols hergestellt werden.

Bei den Verbindungen der Formeln III, IV, V und VII sowie bei den mindestens einen aromatischen Aminrest enthaltenden Pyrrolo[3,4-c]pyrrolen handelt es sich um bekannte Verbindungen. Sollten einige davon noch neu sein, so können sie in Analogie zu allgemein bekannten Verfahren hergestellt werden.

Die Pyrrolo[3,4-c]pyrrole der Formel I eignen sich als Pigmente zum Färben von hochmolekularem organischem Material in derselben Weise wie bereits in den obenerwähnten US-Patenten 4 579 949, 4 778 899 und 4 585 878 ausführlich beschrieben.

Von besonderem Interesse sind die Pyrrolo[3,4-c]pyrrole der Formel I als photoleitfähige Substanzen in elektrophotographischen Photorezeptoren. Solche Photorezeptoren bestehen aus einer leitfähigen Unterlage und einem Photoleiter, der im Dunkeln isolierend ist, aber unter Belichtung leitend wird. Der Photoleiter kann aus einer oder mehreren Schichten bestehen. Im Fall einer einzigen Schicht ist mindestens eine photoleitfähige Substanz in mindestens einem Bindemittel dispergiert oder unmittelbar auf eine leitende Unterlage aufgedampft. Ein mehrschichtiger Photoleiter besteht bevorzugt aus mindestens einer photoleitfähigen Schicht, enthaltend eine oder mehrere photoleitfähige Substanzen, und mindestens einer ladungstransportierenden Schicht.

Ein weiterer Gegenstand der vorliegenden Erfindung ist demnach ein elektrophotographischer Photorezeptor enthaltend mindestens eine leitfähige Unterlage, eine photoleitfähige Schicht und eine ladungstransportierende Schicht, wobei in mindestens einer dieser Schichten mindestens ein Pyrrolo[3,4-c]pyrrol der Formel I und bevorzugt mindestens ein Pyrrolo[3,4-c]pyrrol der Formel II enthalten ist.

Die leitfähige Unterlage kann aus einer Metallplatte oder -folie bestehen, die roh oder z.B durch Aufrauhen vorbehandelt ist und z.B. aus Aluminium, Zink, Magnesium, Kupfer oder einer Legierung dieser Metalle besteht. Im Falle des Aluminiums kann die Vorbehandlung in einem Anodisieren bestehen. Als Unterlagen kommen auch aluminiumbedampfte Kunststoff-Folien sowie Polymerfilme mit metallisierter Oberfläche in Frage.

Der Photoleiter enthält als photoleitfähige Verbindungen mindestens ein Pyrrolo[3,4-c]pyrrol der Formel I und als ladungstransportierende Substanzen Verbindungen, wie z.B. Hydrazone, Oxadiazole, Oxazole oder Pyrazoline, sowie Arylamine, welche im Polymer-Bindemittel gelöst sind. Ein solcher Aufbau erlaubt nach vorgängiger statischer Aufladung und bildmässiger Belichtung die Erzeugung eines entsprechenden Musters aufgeladener und entladener Stellen (latentes Bild), welches nach bekannten Verfahren der Reprographie in ein sichtbares Abbild übergeführt werden kann.

Die Belichtung kann mit Licht im sichtbaren Wellenbereich erfolgen, wobei hohe Lichtempfindlichkeiten erreicht werden.

Zur Erhaltung des statischen Potentials an Stellen, die nicht belichtet werden, tragen die Pyrrolo[3,4-c]pyrrole der Formel I dadurch bei, dass sie einen hohen Dunkelwiderstand aufweisen.

Besteht der Photoleiter aus einer einzigen Schicht, so enthält diese ein oder mehrere Pyrrolo[3,4-c]pyrrole der Formel I zweckmässig in fein verteilter Form, gegebenenfalls zusammen mit ladungstransportierenden Substanzen, in einem organischen Bindemittel. Das Bindemittel ist zweckmässig filmbildend, isolierend und adhäsiv. Je nach Anwendung ist es löslich in organischen Lösungsmitteln oder in basischen Mischungen organischer Lösungsmittel, die gegebenenfalls Wasser enthalten. Besonders geeignet sind Bindemittel auf der Basis von Polykondensations- und Polyadditionsprodukten, wie Polyamiden, Polyurethanen, Polyestern, Epoxyharzen, Phenoxyharzen, Polyketonen, Polycarbonaten, Polyvinylketonen, Polystyrolen, Polyvinylcarbazolen, Polyacrylamiden, Polymethylmethacrylaten, Polyvinylbutyrat, Polyvinylchlorid, Polyvinylacetat sowie Copolymerisaten, wie z.B. Styrol-Maleinsäureanhydrid-Copolymeren, Styrol-Methacrylsäure-Methacrylsäureester-Copolymeren oder Vinylchlorid-Vinylacetat-Copolymeren.

Besteht der Photoleiter aus mehreren Schichten, so sind Doppelschichten von besonderem Interesse. Für diesen Fall wird auf die leitfähige Unterlage zuerst eine photoleitfähige und auf diese eine zweite ladungstransportierende Schicht aufgebracht. Das Aufbringen der Schichten kann auch in umgekehrter Reihenfolge durchgeführt werden. Eine der Schichten, vorzugsweise die ladungserzeugende Schicht, enthält mindestens ein Pyrrolo[3,4-c]-pyrrol der Formel I. Dieses kann in einem organischen Bindemittel gelöst oder fein verteilt sein. Die Applikation auf die leitfähige Unterlage erfolgt beispielsweise durch Auftragen einer Lösung beziehungsweise Dispersion der Bindemittel-Farbkörper-Mischung in einem organischen Lösungsmittel und anschliessendes Verdampfen des Lösungsmittels. Man kann das Pyrrolo[3,4-c]pyrrol der Formel I aber auch auf die leitfähige Unterlage aufdampfen.

Die zweite Schicht enthält eine oder mehrere ladungstransportierende Substanzen, vorzugsweise gelöst oder dispergiert in einem organischen Bindemittel. Als ladungstransportierende Substanzen kommen die verschiedensten aromatischen, vorzugsweise stickstoffhaltigen Verbindungen in Frage, wie Hydrazone oder aromatische Amine, die gegebenenfalls Alkylidenbrücken oder -reste enthalten. Beispielsweise handelt es sich um die im US Patent 4 582 771 auf den Spalten 56-60 sowie in der Publikation "Japan, Hardcopy '88, Post-International Symposium in Kansa; Recent Progress in Hardcopy Materials in Electrophotography", Seite 22 (Osaka, JP, 23.5.88) beschriebenen Substanzen.

Ein weiterer Gegenstand der Erfindung betrifft demnach die Herstellung eines elektrophotographischen Photorezeptors, dadurch gekennzeichnet, dass man ein Pyrrolo[3,4-c]-pyrrol der Formel I mit einem organischen Bindemittel auf eine leitende Unterlage aufträgt oder im Vakuum aufdampft und anschliessend eine zweite ladungstransportierende Schicht, enthaltend eine aromatische, stickstoffhaltige Verbindung, aufbaut.

Zur Verbesserung der physikalischen Eigenschaften der Schichten können sowohl die photoleitfähige als auch die ladungstransportierende Schicht noch Zusätze, wie Egalisiermittel, oberflächenaktive Mittel oder Weichmacher, enthalten.

Die nachstehenden Beispiele erläutern die Erfindung:

Beispiel 1: 6,90 g Natrium werden bei Rückflusstemperatur in 120 ml tert.-Amylalkohol bis zur vollständigen Umsetzung gerührt. Man kühlt auf 90°C ab, fügt 24,02 g 4-Cyanpyridin-N-oxid zu und tropft innerhalb von 4 Stunden 20,23 g Bernsteinsäure-diisopropylester zu. Das Reaktionsgemisch wird 4 Stunden bei 80°C gerührt und anschliessend auf 30°C abgekühlt. Man lässt eine Mischung von 29,56 g Salzsäure und 240 ml Methanol/Wasser (1:2) zulaufen und rührt eine weitere Stunde bei Raumtemperatur. Das ausgefallene Pigment wird abfiltriert, mit Wasser gewaschen und mit Methanol heiss extrahiert. Man filtriert und wäscht die erhaltenen Kristalle mit Methanol und Wasser. Nach Trocknung im Vakuum werden 6,80 g (21 % d. Th.) der Verbindung der Formel 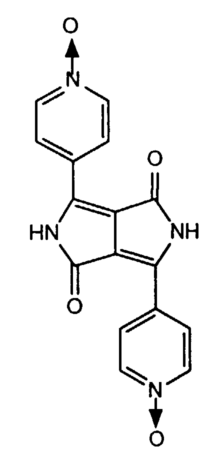 in Form dunkelroter Kristalle mit Schmelzpunkt >250°C isoliert.

| Analyse | C | H | N |
|---|---|---|---|
| Berechnet: | 59,63 % | 3,13 % | 17,38 % |
| Gefunden: | 59,98 % | 4,00 % | 17,82 %. |

Beispiel 2: 6,81 g Natriummethylat werden bei Rückflusstemperatur in 70 ml Methanol bis zur vollständigen Umsetzung gerührt. Man gibt 6,05 g 4-Cyan-pyridin-N-oxid und anschliessend über 2 Stunden in kleinen Portionen 9,71 g der Verbindung der Formel 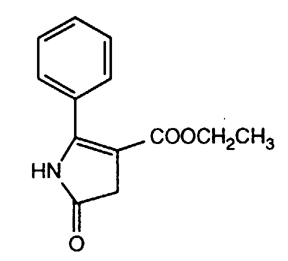 zu. Das Gemisch wird während 4 Stunden am Rückfluss gekocht und darauf auf 40°C abgekühlt. Man lässt eine Mischung von 12,4 g Salzsäure und 140 ml Wasser zutropfen und rührt eine weitere Stunde. Das ausgefallene Pigment wird abfiltriert, mit Wasser gewaschen und mit Methanol heiss extrahiert. Man filtriert und wäscht die erhaltenen Kristalle mit Methanol und dann mit Wasser. Nach der Trocknung im Vakuum bei 80°C erhält man 3,65 g (28,4 % d. Th.) eines Pigmentes der Formel

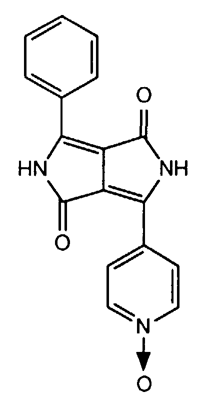

| Analyse | C | H | N |
|---|---|---|---|
| Berechnet: | 66,88 % | 3,63 % | 13,76 % |
| Gefunden: | 66,02 % | 3,96 % | 13,46 %. |

Beispiel 3A: 5,21 g 3-Cyan-pyridin werden unter Rühren in 50 ml Eisessig bei 40°C vollständig gelöst. Dann gibt man 9,8 ml einer 30%igen wässrigen Lösung H₂O₂ zu, erhitzt das Gemisch unter Rühren auf 100°C für 2 Stunden und kontrolliert mittels Dünnschichtchromatographie, dass die Umsetzung vollständig ist Man dampft dann das Lösungsmittel vorsichtig ein, behandelt den beigen Festkörper mit 25 ml Ethanol, kühlt ab und filtriert. Der Rückstand wird bei 35°C unter Vakuum getrocknet. Man erhält 3,5 g (58,3% d. Th.) 3-Cyan-pyridin-N-oxid als weisser Festkörper vom Schmelzpunkt 172-174°C.

| Analyse | C | H | N |
|---|---|---|---|
| Berechnet: | 60,00 % | 3,36 % | 23,32 % |
| Gefunden: | 59,36 % | 3,43 % | 23,07 %. |

Beispiel 3B: 4,18 g Natriummethylat werden in 35 ml über Molekularsieb getrocknetem Methanol unter Stickstoff bei 60°C suspendiert. Man gibt 3,4 g 3-Cyan-pyridin-N-oxid und anschliessend über 1 Stunde in kleinen Portionen 5,78 g der Verbindung der Formel 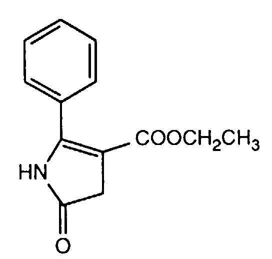 zu. Das Gemisch wird während 5 Stunden am Rückfluss gekocht, dann in eine Lösung von 7,4 g Salzsäure in 70 ml Wasser eingetragen. Die Suspension wird eine Stunde gerührt und abfiltriert. Der Rückstand wird mit Wasser gewaschen und im Vakuum bei 60°C getrocknet. Man erhält 2,1 g (27,5 % d. Th.) eines dunkelroten Pigments der Formel

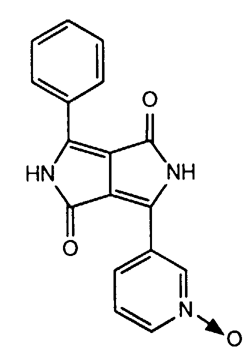

| Analyse | C | H | N |
|---|---|---|---|
| Berechnet: | 66,88 % | 3,63 % | 13,76 % |
| Gefunden: | 66,24 % | 3,71 % | 13,60 %. |

Beispiel 4: 0,3 g des Produktes von Beispiel 2 werden in einem Gemisch von 10 g Xylol und Ethylenglykolmonomethylether (2:1 in Volumen), enthaltend 1,0 g eines handelsüblichen Alkyd/Melaminharz (1:1 in Gewicht), aufgenommen. Die Suspension wird dann während 5 Stunden mit Glaskugeln gemahlen und anschliessend auf eine Aluminiumplatte mit einem Ziehstab aufgetragen (=ladungserzeugende Schicht). Diese Schicht wird bei 50°C während 3 Stunden getrocknet. Die Schichtdicke beträgt ca. 1 µm. Eine zweite Schicht, bestehend aus einem Gemisch von 0,6 g eines Hydrazons der Formel 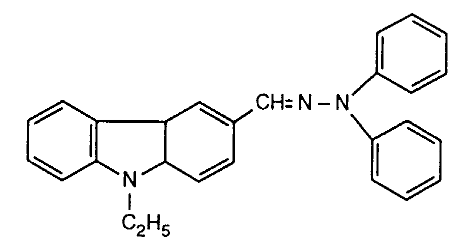 und 0,9 g des Polyacrylatlackes ®Lucite 41 in 11 g Methylethylketon, wird dann aufgetragen und bei 50°C während 15 Stunden getrocknet. Die Schichtdicke beträgt 1 bis 15 µm. Dieses Photorezeptor zeichnet sich durch unerwartet hohe Lichtempfindlichkeit und Aufladbarkeit aus.

Beispiel 5: Das Produkt von Beispiel 2 wird mit einer Geschwindigkeit von 5 Å/Sek. unter einem Vakuum von 10⁻⁶ mbar auf eine Aluminium-Unterlage aufgedampft. Die erhaltene Schichtdicke beträgt ca. 1000 Å. Eine zweite Schicht, bestehend aus einem Gemisch von 0,6 g eines Hydrazons der Formel 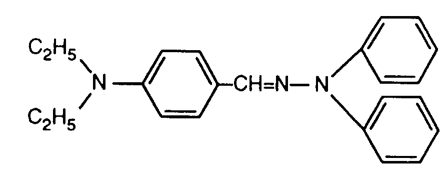 und 0,6 g Polycarbonat (®Makrolon, DuPont) in 10 g Tetrahydrofuran, wird anschliessend aufgetragen und bei 50°C während 6 Stunden getrocknet. Die Schichtdicke beträgt ca. 15 µm. Dieser Photorezeptor zeichnet sich ebenfalls durch eine unerwartet hohe Lichtempfindlichkeit aus.

## Patentansprüche

1. Pyrrolo[3,4-c]pyrrole der Formel 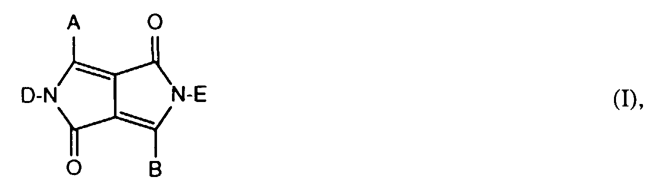 worin A und B unabhängig voneinander für eine Gruppe der Formel 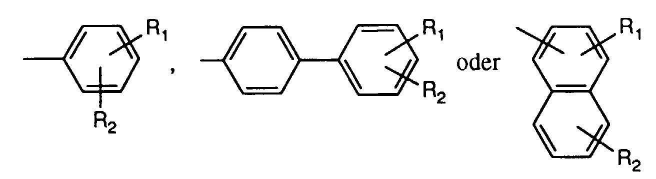 oder für Q stehen, worin
Q einen unsubstituierten oder ein-, zwei- oder dreimal durch C₁-C₁₈-Alkyl, C₁-C₁₈-Alkoxy oder 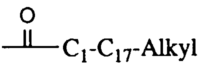 substituierten 5- oder 6-gliedrigen heterocyclischen aromatischen Aminoxidrest ohne oder mit einem oder zwei ankondensierten Benzolringen bedeutet,
R₁ und R₂ unabhängig voneinander Wasserstoff, Halogen, C₁-C₁₈-Alkyl, C₁-C₁₈-Alkoxy, C₁-C₁₈-Alkylmercapto, -CN, -NO₂ oder Trifluormethyl sind,
D und E unabhängig voneinander Wasserstoff, C₁-C₁₈-Alkyl, C₂-C₄-Alkenyl, C₇-C₁₀-Aralkyl, unsubstituiertes oder durch Chlor, Brom, C₁-C₆-Alkyl, C₁-C₄-Alkoxy, Trifluormethyl oder Nitro substituiertes Phenyl bedeuten,
mit der Massgabe, dass mindestens einer der Sustituenten A und B einen Aminoxidest Q bedeutet.

2. Pyrrolo[3,4-c]pyrrole gemäss Anspruch 1, dadurch gekennzeichnet, dass Q ein Rest der Formel 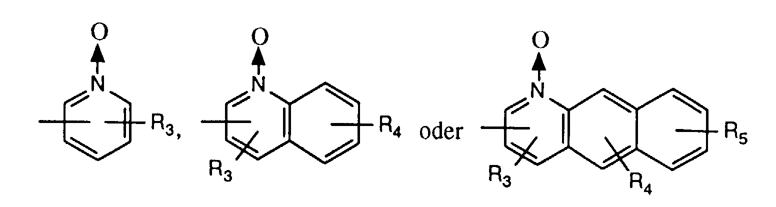 ist, worin R₃, R₄ und R₅ unabhängig voneinander Wasserstoff, C₁-C₄-Alkyl, C₁-C₄-Alkoxy oder 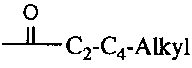 bedeuten.

3. Pyrrolo[3,4-c]pyrrole gemäss Anspruch 2 der Formel 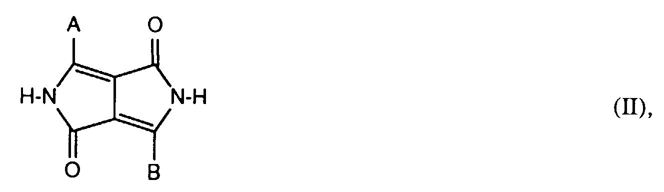 worin A und B unabhängig voneinander für eine Gruppe der Formel 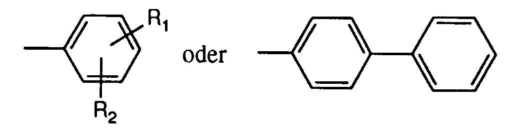 oder für Q stehen, worin
R₁ und R₂ unabhängig voneinander Wasserstoff, Chlor, Brom, C₁-C₄-Alkyl, C₁-C₆-Alkoxy oder CN bedeuten.

4. Pyrrolo[3,4-c]pyrrole gemäss Anspruch 3, dadurch gekennzeichnet, dass Q ein Rest der Formel 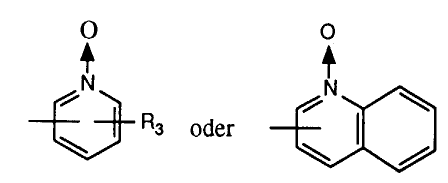 ist, worin R₃ Wasserstoff oder C₁-C₄-Alkyl bedeutet.

5. Verfahren zur Herstellung eines Pyrrolo[3,4-c]pyrrols der Formel I gemäss Anspruch 1, durch Umsetzung von 1 Mol eines Bernsteinsäurediesters der Formel 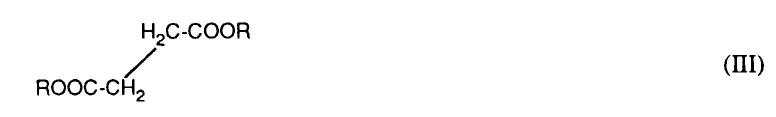 mit je einem Mol der Nitrile 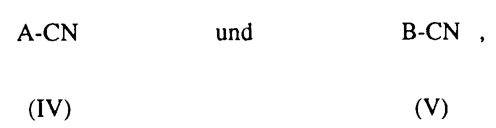 worin R C₁-C₆-Alkyl ist und A und B die in Anspruch 1 angegebene Bedeutung haben, wobei mindestens einer der Reste A und B Q sein muss,
zum Diketopyrrolo[3,4-c]pyrrol der Formel 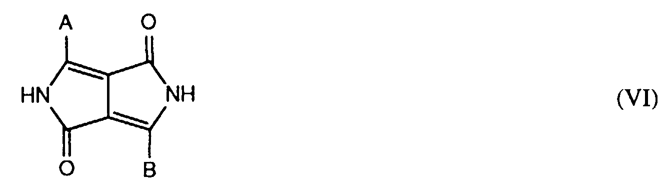 und, wenn D und E in Formel I nicht Wasserstoff bedeuten, durch weitere Umsetzung von 1 Mol des Diketopyrrolo[3,4-c]pyrrols der Formel VI mit je einem Mol einer Verbindung enthaltend die Reste D und E mit der in Anspruch 1 ausser Wasserstoff angegebenen Bedeutung als Abgangsgruppen.

6. Hochmolekulares organisches Material enthaltend ein Pyrrolo[3,4-c]pyrrol der Formel I gemäss Anspruch 1.

7. Elektrophotographischer Photorezeptor enthaltend mindestens eine leitfähige Unterlage, eine photoleitfähige Schicht und eine ladungstransportierende Schicht, dadurch gekennzeichnet, dass in mindestens einer dieser Schichten mindestens ein wie in Anspruch 1 definiertes Pyrrolo[3,4-c]pyrrol der Formel I enthalten ist.

8. Elektrophotographischer Photorezeptor gemäss Anspruch 7, dadurch gekennzeichnet, dass die photoleitfähige Schicht mindestens ein wie in Anspruch 3 definiertes Pyrrolo-[3,4-c]pyrrol der Formel II enthält.

9. Verfahren zur Herstellung eines elektrophotographischen Photorezeptors, dadurch gekennzeichnet, dass man ein wie in Anspruch 1 definiertes Pyrrolo[3,4-c]pyrrol der Formel I mit einem organischen Bindemittel auf eine leitende Unterlage aufträgt oder im Vakuum aufdampft und anschliessend eine zweite Schicht, enthaltend eine aromatische stickstoffhaltige Verbindung, aufbaut.

## Claims

1. A pyrrolo[3,4-c]pyrrole of formula 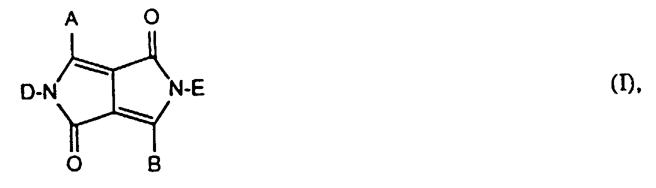 wherein A and B are each independently of the other a group of formula 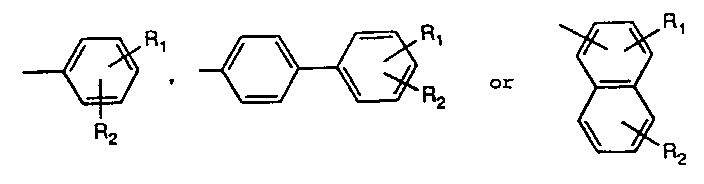 or Q, wherein
Q is a 5- or 6-membered heterocyclic aromatic amine oxide radical which is unsubstituted or substituted by one, two or three C₁-C₁₈alkyl, C₁-C₁₈alkoxy or 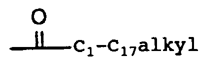 groups and which may carry one or two fused-on benzene rings,
R₁ and R₂ are each independently of the other hydrogen, halogen, C₁-C₁₈alkyl, C₁-C₁₈alkoxy, C₁-C₁₈alkylmercapto, -CN, -NO₂ or trifluoromethyl,
D and E are each independently of the other hydrogen, C₁-C₁₈alkyl, C₂-C₄alkenyl, C₇-C₁₀aralkyl, unsubstituted phenyl or phenyl which is substituted by chloro, bromo, C₁-C₆alkyl, C₁-C₄alkoxy, trifluoromethyl or nitro,
with the proviso that at least one of the substituents A and B is an amine oxide radical Q.

2. A pyrrolo[3,4-c]pyrrole according to claim 1, wherein Q is a radical of formula 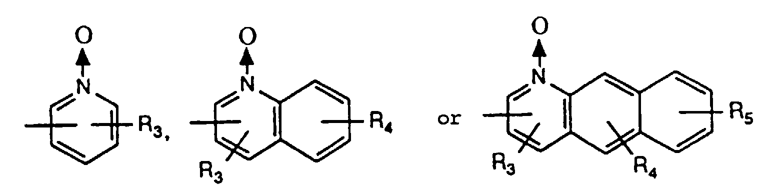 wherein R₃, R₄ and R₅ are each independently of the other hydrogen, C₁-C₄alkyl, C₁-C₄alkoxy or

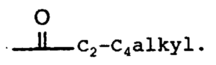

3. A pyrrolo[3,4-c]pyrrole according to claim 2, of formula 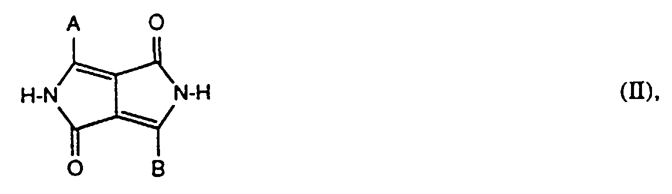 wherein A and B are each independently of the other a group of formula 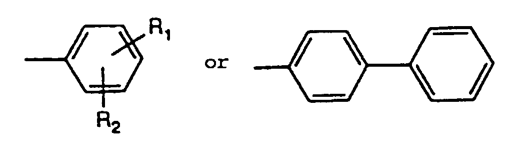 or Q, wherein
R₁ and R₂ are each independently of the other hydrogen, chloro, bromo, C₁-C₄alkyl, C₁-C₆alkoxy or CN.

4. A pyrrolo[3,4-c]pyrrole according to claim 3, wherein Q is a radical of formula 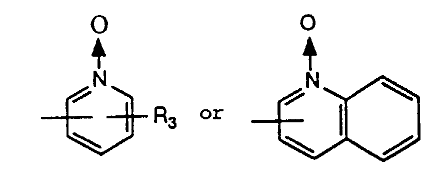 wherein R₃ is hydrogen or C₁-C₄alkyl.

5. A process for the preparation of a pyrrolo[3,4-c]pyrrole of formula I according to claim 1, by reacting 1 mol of a succinic acid diester of formula 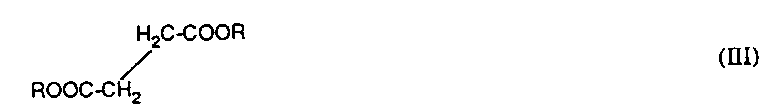 with one mole of each of the nitriles 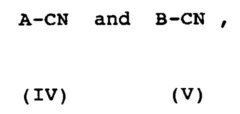 wherein R is C₁-C₆alkyl and A and B are as defined in claim 1, with the proviso that at least one of the radicals A and B must be Q,
to give the diketopyrrolo[3,4-c]pyrrole of formula 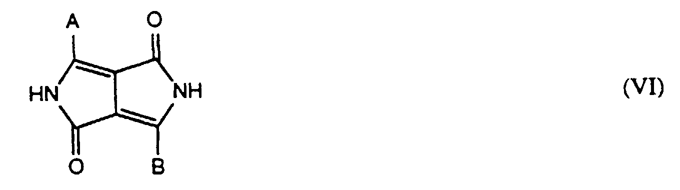 and, if D and E in formula I are not hydrogen, by further reacting 1 mol of the diketopyrrolo[3,4-c]pyrrole of formula VI with in each case one mole of a compound containing the radicals D and E having the signification given in claim 1, with the exception of hydrogen, as leaving groups.

6. organic material of high molecular weight comprising a pyrrolo[3,4-c]pyrrole of formula I according to claim 1.

7. An electrophotograhic photoreceptor comprising at least one conductive substrate, a photoconductive layer and a charge-carrying layer, wherein at least one of these layers contains at least one pyrrolo[3,4-c]pyrrole of formula I as defined in claim 1.

8. An electrophotographic photoreceptor according to claim 7, wherein the photoconductive layer contains at least one pyrrolo[3,4-c]pyrrole of formula II as defined in claim 3.

9. A process for the production of an electrophotographic photoreceptor, which comprises applying a pyrrolo[3,4-c]pyrrole of formula I as defined in claim 1 to a conductive substrate with an organic binder or by vapour deposition under vacuum, and subsequently building up a second layer containing an aromatic nitrogen-containing compound.

## Revendications

1. Pyrrolo[3,4-c]pyrroles de formule 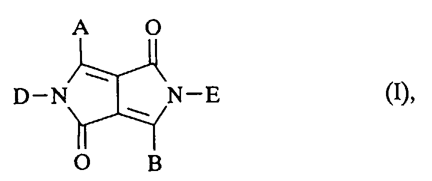 dans laquelle A et B représentent, indépendamment l'un de l'autre, un groupe de formule 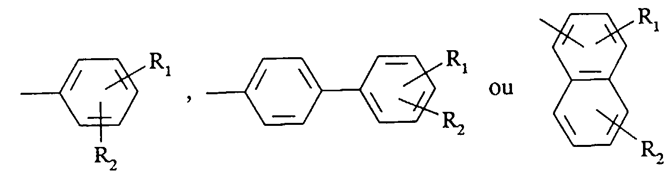 ou un groupe Q, où
Q représente un reste d'oxyde d'amine aromatique hétérocyclique de 5 ou 6 chaînons non substitué ou substitué une, deux ou trois fois par alkyle en C₁-C₁₈, alcoxy en C₁-C₁₈ ou 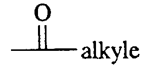 en C₁-C₁₇, sans noyau benzène condensé ou avec un ou deux noyaux benzène condensés,
R₁ et R₂ sont indépendamment l'un de l'autre un atome d'hydrogène ou d'halogène ou un groupe alkyle en C₁-C₁₈, alcoxy en C₁-C₁₈, alkylmercapto en C₁-C₁₈, -CN, -NO₂ ou trifluorométhyle,
D et E représentent indépendamment l'un de l'autre un atome d'hydrogène ou un groupe alkyle en C₁-C₁₈, alcényle en C₂-C₄, aralkyle en C₇-C₁₀, phényle non substitué ou substitué par des restes chloro, bromo, alkyle en C₁-C₆, alcoxy en C₁-C₄, trifluorométhyle ou nitro,
à condition qu'au moins l'un des substituants A et B représente un reste d'oxyde d'amine Q.

2. Pyrrolo[3,4-c]pyrroles selon la revendication 1, caractérisés en ce que Q est un reste de formule 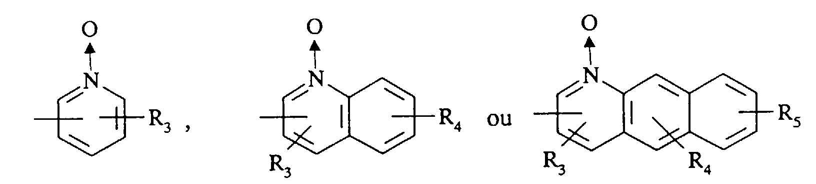 où R₃, R₄ et R₅ représentent indépendamment les uns des autres un atome d'hydrogène ou un groupe alkyle en C₁-C₄, alcoxy en C₁-C₄ ou 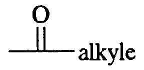 en C₂-C₄.

3. Pyrrolo[3,4-c]pyrroles selon la revendication 2, de formule 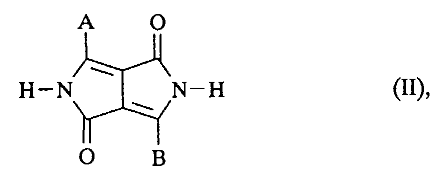 dans laquelle A et B représentent, indépendamment l'un de l'autre, un groupe de formule 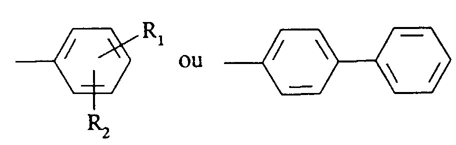 ou un groupe Q, où
R₁ et R₂ représentent indépendamment l'un de l'autre un atome d'hydrogène, de chlore ou de brome ou un groupe alkyle en C₁-C₄, alcoxy en C₁-C₆ ou CN.

4. Pyrrolo[3,4-c]pyrroles selon la revendication 3, caractérisés en ce que Q est un reste de formule 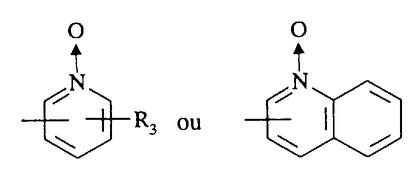 où R₃ représente un atome d'hydrogène ou un groupe alkyle en C₁-C₄.

5. Procédé de préparation d'un pyrrolo[3,4-c]pyrrole de formule I selon la revendication 1, selon lequel on fait réagir 1 mole d'un diester d'acide succinique de formule 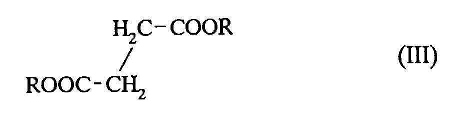 avec une mole de chacun des nitriles
A-CN (IV) et B-CN (V),
où R est un groupe alkyle en C₁-C₆ et A et B ont la signification donnée dans la revendication 1, au moins l'un des A et B devant être un groupe Q,
pour former le dicétopyrrolo[3,4-c]pyrrole de formule 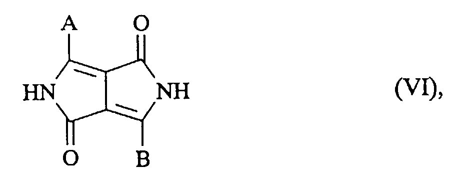 et, lorsque D et E dans la formule I ne représentent pas l'hydrogène, on fait ensuite réagir 1 mole du dicétopyrrolo[3,4-c]pyrrole de formule VI avec à chaque fois 1 mole d'un composé contenant comme groupes partants les restes D et E ayant la signification indiquée dans la revendication 1 à l'exception de l'hydrogène.

6. Matière organique macromoléculaire contenant un pyrrolo[3,4-c]pyrrole de formule I selon la revendication 1.

7. Récepteur photoélectrique électrophotographique contenant au moins un support conducteur, une couche photoconductrice et une couche porteuse de charge, caractérisé en ce qu'au moins une de ces couches contient au moins un pyrrolo[3,4-c]pyrrole de formule I tel que défini dans la revendication 1.

8. Récepteur photoélectrique électrophotographique selon la revendication 7, caractérisé en ce que la couche photoconductrice contient au moins un pyrrolo[3,4-c]pyrrole de formule II tel que défini dans la revendication 3.

9. Procédé de production d'un récepteur photoélectrique électrophotographique, caractérisé en ce que l'on applique ou on vaporise sous vide un pyrrolo[3,4-c]pyrrole de formule I tel que défini dans la revendication 1 avec un liant organique sur un support conducteur, puis on constitue par-dessus une seconde couche contenant un composé aromatique azoté.
